(19)

**Europäisches Patentamt**

**European Patent Office**

**Office européen des brevets**

(11) **EP 3 945 317 A1**

(12) **EUROPEAN PATENT APPLICATION**

(43) Date of publication:
**02.02.2022 Bulletin 2022/05**

(21) Application number: **21188664.3**

(22) Date of filing: **30.07.2021**

(51) International Patent Classification (IPC):
**G01N 33/36** $^{(2006.01)}$

(52) Cooperative Patent Classification (CPC):
**G01N 33/367**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **31.07.2020 IT 202000018730**

(71) Applicants:
- **Avella, Maurizio**
  **80129 Napoli (IT)**
- **Cocca, Mariacristina**
  **84087 Sarno (IT)**
- **De Falco, Francesca**
  **Bacoli (IT)**
- **Di Pace, Emilia**
  **80077 Ischia (IT)**
- **Errico, Maria Emanuela**
  **80129 Napoli (IT)**
- **Gentile, Gennaro**
  **84013 Cava de' Tirreni (IT)**

(72) Inventors:
- **AVELLA, Maurizio**
  **80129 Napoli (IT)**
- **COCCA, Mariacristina**
  **84087 Sarno (IT)**
- **DE FALCO, Francesca**
  **Bacoli (IT)**
- **DI PACE, Emilia**
  **80077 Ischia (IT)**
- **ERRICO, Maria Emanuela**
  **80129 Napoli (IT)**
- **GENTILE, Gennaro**
  **84013 Cava de' Tirreni (IT)**
- **WESTERBOS, Maria**
  **Amsterdam (NL)**

(74) Representative: **Trentin, Michele**
**Eureka IP Consulting**
**Via Giovanni Lanza, 40-44**
**36100 Vicenza (IT)**

(54) **MICRO-FIBER QUANTIFICATION METHOD**

(57) Micro-fiber quantification method released from the washing of at least one fabric comprising at least one simulation test (2) and at least one full-scale washing test (3). Said simulation test (2) comprises a step in which at least one first washing of at least one first fabric sample is performed, together with a first washing solution, subsequently filtered by at least one first filter. The simulation test (2) also comprises counting the quantity of released microfibres, preparing a plurality of number ranges of released microfibres, of which at least a first set is associated with a corresponding plurality of main release levels. Finally, there is a comparison of the counted quantity with the plurality of number ranges to assign a first corresponding release level. The full-scale test (3) comprises performing at least one second washing of at least one second fabric sample with a second washing solution and filtering by at least one second filter. Such a washing test (3) also comprises detecting a weight quantity of the released microfibres, preparing a plurality of weight quantity ranges, of which at least one second set of ranges is associated with the plurality of main release levels, and comparing the detected quantity with the plurality of quantity ranges to assign a second corresponding release level. Such a quantification method also comprises a step of estimating (5) an official release level corresponding to the highest one among the first and the second corresponding release level.

EP 3 945 317 A1

## Description

In the present patent, the term "microfibres" refers to textile fibres, of any chemical composition, having dimensions less than 5 mm.

Furthermore, the term "maximum range" refers to the greatest number range of released microfibres and the weight quantity range of released microfibres.

### Field of application

The present invention is applicable to the textile field and, in particular, relates to the washing of fabrics.

More in detail, the present invention relates to a method for quantifying the microfibres released from the washing of fabrics.

### Background art

In the textile field, in particular, the study of the release of microfibres into the environment, following the washing of the fabrics themselves, plays an important role in the field of fabric treatment, as it is considered one of the main sources of the global release of microplastics into the oceans.

The presence of microfibres, which represent an important subcategory of environmental pollution from microplastics, has been found in several aquatic environments, from rivers to seas and oceans, and in remote environments such as the Arctic and deep waters. In such environments and in other aquatic ecosystems, not only synthetic (e.g., polyester, polyamide, etc.) but also natural (e.g., cotton, wool, etc.) and artificial (e.g., rayon, viscose, etc.) microfibres have been found.

Furthermore, the problems arising from the dispersion of microfibres in the environment are also linked to their coexistence with different marine organisms; in fact, the ingestion of microfibres has been reported for different organisms, from plankton to molluscs, fish and turtles, demonstrating how they are able to persist in the aquatic environment.

According to the above, the dispersion of such textile fibres into the environment appears to be particularly polluting, as, for example, during production and processing they undergo chemical treatments which introduce harmful chemicals into the environment (e.g., dyes, additives, finishing, etc.), which can alter the degradability of the natural fibres in the marine environment.

In addition, the presence of microfibres in aquatic ecosystems may cause the release of harmful substances present therein or may lead to the accumulation on the surface thereof pollutants already present in the surrounding environment.

On closer inspection, regardless of whether the microfibres come from natural, artificial or synthetic fabrics, they are a pollutant for the environment not only because of the nature thereof, but also because of the chemicals linked thereto during the production and processing processes.

Laboratory tests and full-scale tests exist in the prior art for evaluating the microfibre content released by the various fabrics into the environment following their washing.

The laboratory simulations conducted on fabric samples first include their washing and, subsequently, include the filtration of the washing solution, i.e., the cleaning liquids used, so as to evaluate the number of microfibres released.

As far as full-scale washing tests are concerned, the fabric samples are washed in commercial washing machines from which all the wastewater is collected and filtered to assess, again, the quantity of microfibres released.

However, the laboratory simulations subject the fabric samples to greater stress than full-scale washings with the resulting overestimation of the quantity of microfibres released.

Furthermore, generally the counting of the microfibres released in laboratory test washing solutions is conducted according to a statistical sampling and not performing an integral analysis of the filter implying, therefore, a further approximation of the evaluations deriving from the laboratory tests due to times and costs which would involve performing an integral count of the microfibres retained by the filter.

Moreover, differences in laboratory testing performance compared to full-scale analyses, in particular, regarding the differences between the quantity of filtered washing solution and the size of the fabric samples, make it difficult to obtain comparable results therebetween. In fact, there is firstly the need to use filters with different meshes, narrower in the case of laboratory tests and, consequently, a count of the quantity of residual microfibres which produces results which are not comparable to each other. Furthermore, the laboratory tests generally produce results in terms of the number of microfibres released while, given the much larger size of the fabric samples, the full-scale tests produce results in terms of the weight of the microfibres released.

For example, the scientific article entitled "Quantification of microfibres released during washing of synthetic clothes in real conditions and at lab scale" by De Falco Francesca et al. is known and published in "The European physical journal plus", Springer Berlin Heidelberg (2018-07-11), which describes the execution of full-scale and laboratory tests as reported above and, therefore, which have the disadvantages described.

### Presentation of the invention

The object of the present invention is to provide a method for quantifying the microfibres released from the washing of fabrics which allows to at least partially overcome the drawbacks highlighted above.

In particular, an object of the present invention is to provide a quantification method adapted to be used for counting the microfibres released from any type and shape of fabric.

Another object of the present invention is to provide a method for quantifying the microfibres released from the washing of fabrics capable of comparing the results derived from laboratory simulation tests with the results of full-scale tests.

A further object of the present invention is to provide a quantification method which allows to define a release level of the quantity of microfibres characteristic of the type of fabric analysed.

Said objects, as well as others which will become clearer in the following description, are achieved by a micro-fiber quantification method released from the washing of at least one fabric in accordance with the claims which follow, which are to be considered an integral part of the present patent.

In particular, the quantification method of the invention comprises at least one simulation test and at least one full-scale washing test.

According to an aspect of the invention, the simulation test comprises at least one step in which at least a first washing of at least a first fabric sample is performed inside at least one container together with a first washing solution.

Thereafter, there is a step of filtering the first washing solution by at least a first filter with the purpose of retaining the released microfibres and then counting the quantity present on at least a portion of the first filter.

According to another aspect of the invention, the simulation test also comprises a step in which a plurality of number ranges of released microfibres are prepared to then associate with at least a first set of ranges belonging to the plurality of number ranges of released microfibres a corresponding plurality of main release levels.

Finally, the simulation test also comprises a step in which to compare the counted quantity of released microfibres with the plurality of number ranges of released microfibres to assign a first corresponding release level to the counted quantity.

According to a further aspect of the invention, the full-scale washing test comprises a step in which at least a second washing of at least a second fabric sample is performed in at least one washing machine with a second washing solution.

Following such a second washing, the full-scale test comprises a step of filtering the second washing solution by at least a second filter to retain the released microfibres and also a step of detecting a quantity by weight of the released microfibres present in the second filter.

Thereafter, a plurality of weight quantity ranges of released microfibres are prepared and associated with at least a second set of ranges belonging to the plurality of weight quantity ranges of the plurality of main release levels.

In addition, the full-scale washing test also comprises a step of comparing the detected quantity of microfibres released with the plurality of weight quantity ranges of microfibres released to assign a second corresponding release level among the main release levels to the second quantity detected.

According to another aspect of the invention, the quantification method then comprises a step of estimating an official release level corresponding to the highest among the first corresponding release level and the second corresponding release level.

Advantageously, the quantification method of the invention is adapted to be used regardless of the morphological features of the fabric from which the first and the second sample are obtained.

On closer inspection, the quantification method of the invention allows both the first and the second sample to be assigned a release level such that the results of the simulation test are, still advantageously, comparable with the results of the full-scale washing test.

Furthermore, from the aforementioned comparison, the quantification method allows to estimate an official release level characteristic of the type of fabric constituting the first and the second sample analysed.

According to a further aspect of the invention, the method for quantifying microfibres released from the washing of a fabric also comprises a step of preparing a plurality of intermediate release levels among pairs of main release levels. Each of said intermediate release levels corresponding to one of the plurality of number ranges of released microfibres and one of the plurality of weight quantity ranges of released microfibres.

Advantageously, the introduction of the plurality of intermediate release levels allows the method of the invention to increase the accuracy in defining relative number ranges of microfibres and weight quantity ranges, thereby improving accuracy in defining the official release level.

According to another aspect of the invention, following the preparation of the plurality of intermediate release levels, the step of comparing the counted quantity of released microfibres with the plurality of number ranges of released microfibres assigns the counted quantity a first corresponding release level among the main and intermediate release levels.

Similarly, the step of comparing the detected quantity of released microfibres to the plurality of weight quantity ranges of released microfibres assigns a second corresponding release level among the main and intermediate release levels to the detected quantity.

Consequently, the estimation step of the official release level also considers the presence of the aforementioned intermediate release levels. In fact, if the first corresponding release level and the second corresponding release level are consecutive and one among the first corresponding release level and the second corresponding release level corresponds to one among the intermediate release levels and the other corresponds to one among the main release levels, the official release level corresponds to the latter.

Furthermore, if the first corresponding release level and the second corresponding release level are not consecutive and one among them corresponds to one of the intermediate release levels and another corresponds to one among the main release levels, the official release level corresponds to the other if the first corresponding release level is lower than the second corresponding release level, otherwise the official release level corresponds to the main release level immediately preceding the first corresponding release level.

Furthermore, if both of the corresponding release levels correspond to the same intermediate release level, the official release level corresponds to the first among the main release levels above such an intermediate release level.

Otherwise, if the first and second corresponding release levels correspond to two consecutive intermediate release levels, the official release level corresponds to the main release level interposed between the two consecutive intermediate release levels.

As mentioned, the use of intermediate release levels allows to improve accuracy in assigning the official release level.

Furthermore, advantageously, the comparison for the assignment of the official release level evaluates the number range and the weight quantity range of released microfibres in a weighted manner. In fact, the presence of intermediate levels allows to "weight" the released quantity obtained from the two tests.

## Brief description of the drawings

Further features and advantages of the invention will become more evident in light of the detailed description of a preferred but non-exclusive embodiment of a quantification method according to the invention, illustrated by way of nonlimiting example with the aid of the accompanying drawings, in which:

FIG. 1 depicts a block diagram of the quantification method according to the invention;
FIG. 2 depicts a table showing indications on the assignment of the official release level according to the method of FIG. 1.
FIG. 3 depicts a table showing indications on the assignment of the official release level according to an alternative embodiment of the method of the invention.

## Detailed description of some preferred embodiments

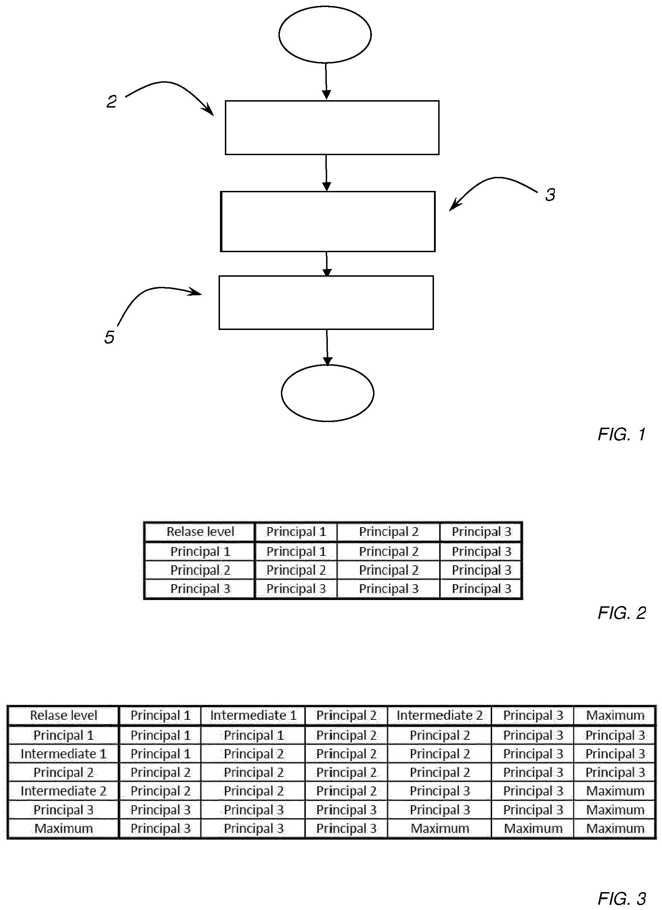

With reference to the above-mentioned drawings, and in particular fig. 1, a micro-fiber quantification method released from the washing of a fabric according to the invention is described.

In particular, the method of the invention comprises a simulation test, indicated in the aforementioned fig.1 with the number **2**, and a full-scale washing test, also indicated in fig. 1 with the number **3**.

The simulation test **2**, i.e., an experimental test generally conducted by means of a laboratory simulator, firstly comprises a step in which a first fabric sample is washed inside a container together with a first washing solution. The latter typically comprises, but is not necessarily limited to, anionic and non-ionic surfactants, soap and phosphates, whitening agents, enzymes, and fragrance. The shape and dimensions of the first sample are aspects which must not be considered limiting for alternative embodiments of the invention.

Furthermore, according to the embodiment of the invention which is described, the washing step is preceded by a step of inserting one or more balls into the container used for the first washing.

Advantageously, the presence of the balls in contact with the first sample during the washing thereof allow to subject the latter to actions similar to a real washing, or in any case to solicit the loss of microfibres during the test. Obviously, such an aspect should not be considered limiting for embodiments of the invention where, for example, elements of different geometry are inserted inside the container or no element is inserted.

According to another aspect of the invention, the simulation test 2 also comprises a step of filtering the first washing solution by a first filter to retain the microfibres released following the first washing. Obviously, the number of filters must not be considered limiting for alternative embodiments of the invention.

Subsequently, according to the quantification method of the invention the quantity of microfibres released in a portion of the first filter is counted. Furthermore, a plurality of number ranges of released microfibres are prepared to then associate at least a first set of such ranges with a corresponding plurality of main release levels.

According to the embodiment of the invention which is described, the counting of the quantity of microfibres released is performed by electronic microscopy. Obviously, also such an aspect must not be considered limiting for alternative embodiments of the invention where, for example, the counting is performed by optical microscopy.

In particular, according to the embodiment of the invention which is described, the counting is based on a sampling of the surface of the first filter. Advantageously, the sampling count allows to speed up its performance, thus saving time and money.

Obviously, such an aspect must not be considered limiting for alternative embodiments of the invention where, for example, the counting is performed on the entire surface of the first filter.

Finally, the counted quantity of released microfibres is compared with the plurality of number ranges of released microfibres to assign a first corresponding release level among the main release levels thereto.

As regards the full-scale washing test **3**, it comprises a step of performing a second washing with a second washing solution of a second fabric sample by means of a washing machine. Such a second washing solution is typically, but not necessarily, the same as the first washing solution used for the first washing.

After the second washing, according to the full-scale test, such a second washing solution is filtered by a second filter to retain the microfibres released from the second sample.

According to the embodiment of the invention which is described, such a filtration of the second washing solution is performed by several filters having decreasing mesh.

Advantageously, the use of multiple filters allows the second washing solution to be screened more efficiently, especially considering the large quantity of waste fluids produced by the washing by means of a washing machine. Obviously, also the number of filters used to filter the second washing solution must not be considered limiting for different alternative embodiments of the invention.

According to a further aspect of the invention, the full-scale test **3** comprises a step in which the weight quantity of the released microfibres present in said second filter is detected and also a step in which a plurality of weight quantity ranges of released microfibres is prepared of which a second set is associated with the plurality of main release levels.

According to the embodiment of the invention which is described, the detection of the weight quantity of the released microfibres present in the second filter is performed by means of a gravimetric analysis of the second filter. Obviously, the weighing technique of the second filter must not be considered as limiting for alternative embodiments of the invention.

Furthermore, it also comprises a comparison of the detected quantity with the plurality of weight quantity ranges of released microfibres to assign to such a detected quantity a second corresponding release level among the main release levels.

According to another aspect of the invention, the quantification method also comprises a step of estimating an official release level **5** as observable in fig. 2 where the rows and columns have the main release levels respectively associated with the counted quantity of released microfibres and the weight quantities of released microfibres.

Such an estimation step **5** is conducted by matching the official release level with the highest among the first corresponding release level and the second corresponding release level.

Advantageously, the quantification method of the invention allows to evaluate the microfibres released during the washing regardless of the type of fabric used to make the first and the second sample.

Furthermore, the method of the invention still advantageously allows to compare the release levels resulting from different tests. In particular, it allows to compare a first corresponding release level with a second corresponding release level resulting respectively from a simulation test and a full-scale washing test.

Still advantageously, the quantification method of the invention allows to estimate an official release level describing the release of the quantity of microfibres characteristic of the type of fabric analysed.

According to an alternative of the embodiment of the invention which is described, the quantification method also comprises a step of preparing a plurality of intermediate release levels, among pairs of main release levels, each of which corresponds to one of the plurality of number ranges and to one of the plurality of weight quantity ranges of released microfibres.

Advantageously, the use of intermediate release levels in the quantification method allows to improve the matching between the ranges of numbers and the weight quantities of the microfibres released and the respective release levels.

According to another aspect of the invention, the step of comparing the counted quantity of released microfibres with the plurality of number ranges of released microfibre assigns a first corresponding release level thereto among the main and intermediate release levels.

Similarly, the step of comparing the detected quantity of microfibres released with the plurality of weight quantity ranges of microfibres released assigns a second corresponding release level to said detected quantity among the main and intermediate release levels.

In addition, the step of estimating the official release level of the method of the invention, against the introduction of intermediate release levels, includes that if the first corresponding release level and the second corresponding release level are consecutive and one among the first corresponding release level and the second corresponding release level corresponds to an intermediate release level and another among the first corresponding release level and the second corresponding release level corresponds to a main release level, the official release level corresponds to the latter.

Furthermore, if the first corresponding release level and the second corresponding release level are not consecutive and one among them corresponds to one of the intermediate release levels and another corresponds to one among the main release levels, the official release level corresponds to the other if the first corresponding release level is lower than the second corresponding release level, otherwise the official release level corresponds to the main release level immediately preceding the first corresponding release level.

Furthermore, if both of the corresponding release levels correspond to the same intermediate release level, the official release level corresponds to the first among the main release levels above the same intermediate release level.

Alternatively, if the first corresponding release level and the second corresponding release level correspond to two consecutive intermediate release levels, the official release level corresponds to the intermediate main release level among such two consecutive intermediate release levels.

The presence of main and intermediate levels advantageously allows to improve the accuracy and precision in estimating the official release level.

Still advantageously, the estimation is performed by evaluating the belonging of corresponding release levels to a group of main and/or intermediate levels so as to "weight" the comparison.

According to the embodiment of the invention which is described, the quantification method also comprises a step of preparing a maximum release level corresponding to a maximum number range of released microfibres and a maximum weight quantity range of released microfibres.

Accordingly, the step of comparing the counted quantity of released microfibres to the plurality of number ranges of released microfibres assigns a first corresponding release level to the counted quantity among the main, intermediate, and maximum release levels. Similarly, comparing the detected quantity with the plurality of weight quantity ranges of released microfibres assigns a second corresponding release level thereto among the main, intermediate, and maximum release levels.

Therefore, considering the maximum release level, the step of estimating the official release level of the quantification method includes, as can be seen in fig. 3 where the rows and columns have the main, intermediate and maximum release levels respectively associated with the counted quantity of microfibres released and the weight quantities of microfibres released, if one among the first corresponding release level and the second corresponding release level corresponds to the maximum release level and another one thereamong corresponds to a main release level immediately preceding said maximum release level, then the official release level corresponds to the maximum release level.

Furthermore, if one among the first and the second corresponding release level corresponds to the maximum release level and another thereamong corresponds to an intermediate release level immediately preceding the maximum release level, the official release level corresponds to the maximum release level.

Otherwise, the official release level corresponds to a main release level immediately preceding the maximum release level.

Advantageously, the maximum release level allows to further improve the accuracy of the quantification method of the invention. Indeed, as in the case of intermediate release levels, the estimate of the official release level conducted considering also a maximum release level is conducted by evaluating the results as a function of the level to which they belong and not simply by performing an arithmetic average.

To further improve the accuracy of the estimation of the official release level, according to different alternative embodiments of the method of the invention, the simulation test and the full-scale washing test are repeated several times, then comparing, for example, an average of the quantity counted and the quantity detected.

In addition, according to different alternative embodiments of the invention, the counted quantity of released microfibres and the detected quantity of released microfibres are respectively expressed by weight of the first sample and the second sample.

In other words, the result obtained from the simulation test is expressed as the counted quantity of microfibres released per, for example, grams of washed fabric and, similarly, the result of the full-scale test is expressed as the detected quantity of microfibres released per kilogram of washed fabric.

Advantageously, expressing the results as mentioned allows to once again improve the estimate of the official release level, making it independent of the size of the first and of the second fabric sample used.

In addition, according to an embodiment of the invention, not shown in the figures, the simulation test also comprises a step of normalizing the counted quantity of released microfibres. In such a step, the release is defined in terms of the number of standard microfibres, for example indicated by N*, released from the first fabric sample tested.

Such a normalization step allows to obtain a normalized parameter for the different morphological features of the microfibres released from different fabric samples (e.g., two different fabrics can release the same number of microfibres but having different sizes) which advantageously allows to improve the evaluation of released microfibres.

Still advantageously, the normalization of the counted quantity allows to further improve the estimation of the official release level.

For example, the normalization step defines a "standard microfibre", a microfibre having length L* and diameter D* and the number N* will indicate the number of standard microfibres (of sizes L* and D*) released from the fabric sample tested, calculated by dividing the total volume of the released microfibres present in the first filter or in a portion thereof, by the volume of the standard microfibre, according to the following formula:

$$N^* = \left(\frac{D}{D^*}\right)^2 \cdot \frac{\sum_i L_i}{L^*}, \text{ i=1, 2, 3, ...}$$

Obviously, the formula used to determine the number of standard microfibres N* must not be considered limiting for different alternative embodiments of the invention.

Regardless of the alternative embodiment being referred to, the simulation test and the full-scale washing test comprise an initial step of pre-washing the respective samples to advantageously clean them of any dirt which may in any manner alter the test results.

In light of the foregoing, it is understood that the method of quantifying the microfibres released from the washing of a fabric of the invention achieves all the intended objects.

In particular, the quantification method of the invention allows the use thereof for counting the microfibres released by any type and shape of fabric.

Furthermore, the method of the invention allows to compare the results of simulation tests, conducted with laboratory simulators, with the results derived from full-scale tests.

Finally, the quantification method of the invention allows to define a release level of the quantity of microfibres characteristic of the type of fabric analysed.

The invention might be subject to many changes and variants, which are all included in the appended claims. Moreover, all the details and steps may furthermore be replaced by other technically equivalent elements, and the materials may be different depending on needs, without departing from the protection scope of the invention defined by the appended claims.

**Claims**

1.  A micro-fiber quantification method released from the washing of at least one fabric comprising at least one simulation test (**2**) and at least one full-scale washing test (**3**), said at least one simulation test (**2**) comprising the following steps:

    — performing at least one first washing of at least one first sample of said at least one fabric within at least one container together with a first washing solution;
    — filtering, after said at least one first washing, said first washing solution by means of at least one first filter to retain said released microfibres;
    — counting the quantity of said released microfibres present on at least a portion of said at least one first filter;
    — preparing a plurality of number ranges of released microfibres and associating with at least a first set of ranges belonging to said plurality of number ranges of released microfibres a corresponding plurality of main release levels;
    — comparing said counted quantity of released microfibres to said plurality of number ranges of released microfibres to assign a first corresponding release level among said main release levels to said counted quantity,

    said at least one full-scale washing test (3) comprising the following steps:

    — performing at least one second washing of at least one second sample of said at least one fabric in at least one washing machine with a second washing solution;
    — filtering, after said at least one second washing, said second washing solution by means of at least one second filter to retain said released microfibres;
    — detecting a quantity by weight of said released microfibres present in said at least one second filter;
    — preparing a plurality of weight quantity ranges of released microfibres and associating said plurality of main release levels with at least one second set of ranges belonging to said plurality of weight ranges;
    — comparing said detected quantity of released microfibres to said plurality of weight quantity ranges of released microfibres to assign a second corresponding release level among said main release levels to said detected quantity,

    said quantification method comprising an estimation step (**5**) of an official release level in which said official release level corresponds to the highest among said first corresponding release level and said second corresponding release level.

2.  Micro-fiber quantification method according to claim 1, comprising a step of preparing a plurality of intermediate release levels among pairs of said main release levels, each of said intermediate release levels corresponding to one of said plurality of number ranges of released microfibres and one of said plurality of weight quantity ranges of released microfibres.

3.  Micro-fiber quantification method according to claim 2, **wherein:**

    — said step of comparing said counted quantity of released microfibres to said plurality of number ranges of released microfibres assigns a first corresponding release level among said main and intermediate release levels to said counted quantity;
    — said step of comparing said detected quantity of released microfibres to said plurality of weight quantity ranges of released microfibres assigns a second corresponding release level among said main and intermediate release levels to said detected quantity;
    — in said step of estimating said official release level:

        ◦ if said first corresponding release level and second corresponding release level are consecutive and one among said first corresponding release level and second corresponding release level corresponds to one of said intermediate release levels and another one among said first corresponding release level and second corresponding release level corresponds to one of said main release levels, said official release level corresponds to said other one among said first corresponding release level and second corresponding release level;

∘ if said first corresponding release level and second corresponding release level are not consecutive and one among said first corresponding release level and second corresponding release level corresponds to one of said intermediate release levels and another one among said first corresponding release level and second corresponding release level corresponds to one of said main release levels, said official release level corresponds to said other one among said first corresponding release level and second corresponding release level if said first corresponding release level is lower than said second corresponding release level, otherwise said official release level corresponds to said main release level immediately preceding said first corresponding release level;

∘ if both said first and second corresponding release levels correspond to the same of said intermediate release levels, said official release level corresponds to the first one among said main release levels greater than said same of said intermediate release levels;

∘ if said first corresponding release level and second corresponding release level correspond to two consecutive among said intermediate release levels, said official release level corresponds to the level among said intermediate main release levels among said two consecutive among said intermediate release levels.

4. Micro-fiber quantification method according to claim 3, **wherein**:

— said method comprises a step of preparing a maximum release level corresponding to a maximum number range of released microfibres and a maximum weight quantity range of released microfibres;

— said step of comparing said counted quantity of released microfibres to said plurality of number ranges of released microfibres assigns a first corresponding release level among said main, intermediate and maximum release levels to said counted quantity;

— said step of comparing said detected quantity of released microfibres to said plurality of weight quantity ranges of released microfibres assigns a second corresponding release level among said main, intermediate and maximum release levels to said detected quantity;

— in said step of estimating said official release level:

∘ if one among said first corresponding release level and second corresponding release level corresponds to said maximum release level and another one among said first corresponding release level and second corresponding release level corresponds to a main release level immediately preceding said maximum release level, said official release level corresponds to said maximum release level;

∘ if one among said first corresponding release level and second corresponding release level corresponds to said maximum release level and another one among said first corresponding release level and second corresponding release level corresponds to an intermediate release level immediately preceding said maximum release level, said official release level corresponds to said maximum release level;

∘ otherwise said official release level corresponds to said main release level immediately preceding said maximum release level.

5. Micro-fiber quantification method according to one or more of the previous claims, **wherein** said counting of said quantity of said released microfibres present on said at least one portion of said at least one first filter in said simulation test is performed by means of electron and/or optical microscopy of said at least one first filter.

6. Micro-fiber quantification method according to one or more of the previous claims, **wherein** said detection of said quantity by weight of said released microfibres present in said at least one second filter is performed by means of a gravimetric analysis of said at least one second filter.

7. Micro-fiber quantification method according to one or more of the previous claims, comprising a step of inserting one or more balls into said at least one container before said at least one first washing.

8. Micro-fiber quantification method according to one or more of the previous claims, **wherein** said at least one simulation test and said at least one full-scale washing test are repeated several times.

**9.** Micro-fiber quantification method according to one or more of the previous claims, **wherein** said counted quantity of released microfibres and said detected quantity of released microfibres are expressed respectively by weight of said at least one first sample and said at least one second sample of said at least one fabric.

**10.** Micro-fiber quantification method according to one or more of the previous claims, **wherein** said filtration, after said at least one second washing, of said second washing solution is performed by means of several filters having decreasing mesh.

*FIG. 1*

| Relase level | Principal 1 | Principal 2 | Principal 3 |
|---|---|---|---|
| Principal 1 | Principal 1 | Principal 2 | Principal 3 |
| Principal 2 | Principal 2 | Principal 2 | Principal 3 |
| Principal 3 | Principal 3 | Principal 3 | Principal 3 |

*FIG. 2*

| Relase level | Principal 1 | Intermediate 1 | Principal 2 | Intermediate 2 | Principal 3 | Maximum |
|---|---|---|---|---|---|---|
| Principal 1 | Principal 1 | Principal 1 | Principal 2 | Principal 2 | Principal 3 | Principal 3 |
| Intermediate 1 | Principal 1 | Principal 2 | Principal 2 | Principal 2 | Principal 3 | Principal 3 |
| Principal 2 | Principal 2 | Principal 2 | Principal 2 | Principal 2 | Principal 3 | Principal 3 |
| Intermediate 2 | Principal 2 | Principal 2 | Principal 2 | Principal 3 | Principal 3 | Maximum |
| Principal 3 | Principal 3 | Principal 3 | Principal 3 | Principal 3 | Principal 3 | Maximum |
| Maximum | Principal 3 | Principal 3 | Principal 3 | Maximum | Maximum | Maximum |

*FIG. 3*

Europäisches
Patentamt

European
Patent Office

Office européen
des brevets

# EUROPEAN SEARCH REPORT

Application Number

EP 21 18 8664

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DE FALCO FRANCESCA ET AL: "Quantification of microfibres released during washing of synthetic clothes in real conditions and at lab scale*", THE EUROPEAN PHYSICAL JOURNAL PLUS, SPRINGER BERLIN HEIDELBERG, BERLIN/HEIDELBERG, vol. 133, no. 7, 11 July 2018 (2018-07-11), pages 1-4, XP036542474, DOI: 10.1140/EPJP/I2018-12123-X * page 2, line 1 - page 4, line 7 * | 1-10 | INV. G01N33/36 |
| A | SILLANPÄÄ MARKUS ET AL: "Release of polyester and cotton fibers from textiles in machine washings", ENVIRONMENTAL SCIENCE AND POLLUTION RESEARCH INTERNATIONAL, ECOMED, LANDSBERG, DE, vol. 24, no. 23, 1 July 2017 (2017-07-01), pages 19313-19321, XP036298497, ISSN: 0944-1344, DOI: 10.1007/S11356-017-9621-1 [retrieved on 2017-07-01] * page 19314, right-hand column, line 34 - page 19316, right-hand column, line 21 * | 1-10 | |
| A | YANG LIBIAO ET AL: "Microfiber release from different fabrics during washing", ENVIRONMENTAL POLLUTION, vol. 249, 8 March 2019 (2019-03-08), pages 136-143, XP085691208, ISSN: 0269-7491, DOI: 10.1016/J.ENVPOL.2019.03.011 * page 137, left-hand column, line 33 - page 139, left-hand column, line 41 * | 1-10 | TECHNICAL FIELDS SEARCHED (IPC) G01N |

-/--

The present search report has been drawn up for all claims

| Place of search | Date of completion of the search | Examiner |
|---|---|---|
| The Hague | 1 September 2021 | Gilow, Christoph |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

| | Europäisches Patentamt European Patent Office Office européen des brevets | **EUROPEAN SEARCH REPORT** | Application Number EP 21 18 8664 |

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | CESA FLAVIA SALVADOR ET AL: "Laundering and textile parameters influence fibers release in household washings", ENVIRONMENTAL POLLUTION, BARKING, GB, vol. 257, 15 November 2019 (2019-11-15), XP085978966, ISSN: 0269-7491, DOI: 10.1016/J.ENVPOL.2019.113553 [retrieved on 2019-11-15] * page 3, left-hand column, line 1 - page 4, right-hand column, line 39 * ----- | 1-10 | |

TECHNICAL FIELDS SEARCHED (IPC)

The present search report has been drawn up for all claims

| Place of search | Date of completion of the search | Examiner |
|---|---|---|
| The Hague | 1 September 2021 | Gilow, Christoph |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- Quantification of microfibres released during washing of synthetic clothes in real conditions and at lab scale. **DE FALCO FRANCESCA et al.** The European physical journal plus. Springer, 11 July 2018